# EUROPÄISCHE PATENTSCHRIFT

(11) **EP 2 337 539 B1**
(45) Veröffentlichungstag und Bekanntmachung des Hinweises auf die Patenterteilung: **25.07.2018**
(21) Anmeldenummer: 09777990.4
(22) Anmeldetag: 20.08.2009
(51) Int. Cl.: A61F 13/02

(54) **WÄRMEPFLASTER**
HOT COMPRESS
PANSEMENT CHAUFFANT

(30) Priorität: 15.09.2008 DE 102008048327
(43) Veröffentlichungstag der Anmeldung: 29.06.2011
(73) Patentinhaber: Beiersdorf AG, 20253 Hamburg (DE)
(72) Erfinder: WÖLLER, Karl-Heinz, 20257 Hamburg (DE); NEMEC, Dominik, 70199 Stuttgart (DE); ERISMIS, Harun, D-70193 Stuttgart (DE)
(86) Internationale Anmeldenummer: PCT/EP2009/006027
(87) Internationale Veröffentlichungsnummer: WO 2010/028740

(56) Entgegenhaltungen:
- EP-A1- 1 782 767
- WO-A2-2005/044141
- WO-A2-2006/122736

## Beschreibung

Wärmeanwendungen zur medizinischen oder kosmetischen Behandlung von Mensch und Tier sind seit der Antike bekannt. Die ursprünglichsten Anwendungsformen sind mit warmen Wasser getränkte Tücher zur Entspannung von Gesichtshaut nach der Rasur, warme Wickel bei Erkältungserkrankungen, Fangopackungen bei Muskelverspannungen oder mit heißem Wasser gefüllte Wärmflaschen bei Magenbeschwerden.

In einer modernen Ausführung kann bei medizinischen Anwendungen am Bewegungsapparat anstatt Letztgenanntem auch ein mit einem wässrigem Gel gefüllter wiederverwendbarer Plastikbeutel, angewendet werden, z.B. Hansaplast® Hot/Cold Pack. Nachteilig an Produkten dieser Art ist, dass die Anwendungsform vorher erwärmt werden muss, z.B. in einem Wasserbad oder in der Mikrowelle und während der Anwendung dann kontinuierlich an Wärme verliert.

Neben vielerlei weiteren Ausprägungsformen und Indikationen der vorgenannten Anwendungen kommen seit Beginn des 20. Jahrhunderts vermehrt selbstklebende Wärmepflaster zum Einsatz. Haupteinsatzgebiete dieser Wärmepflaster sind dabei u.a. Schmerzzustände des Bewegungsapparates, aber auch kosmetische Indikationen wie Narben- oder Cellulitebehandlung.

Die beiden gängigsten Basissysteme von Wärmepflastern sind zum einen solche die pharmazeutisch aktive Substanzen, einen Wirkstoff, in die Haut abgeben und dort physiologisch wirken lassen, z.B. capsaicinoidhaltige Produkte wie z.B. Hansaplast® ABC Wärme-Pflaster. Die zweite Gruppe von Wärmepflastern nutzt exotherme chemische Reaktionen wie zum Beispiel die exotherme Oxidation von Eisen als rein physikalische Wärmequelle, welche die Wärme auf das dem Pflaster unterliegende Gewebe abgibt, z.B. Hansaplast® Therapeutic Heat Pad.

Nachteilig bei Pflastern mit einem Wirkstoff zur Wärmeempfindung ist, dass erst eine gewisse Zeit vergehen muss bis eine wirksame Menge des jeweiligen Wirkstoffs aus der Pflastermatrix in die Haut penetriert ist. Auch ist das Wärmeempfinden von Anwender zu Anwender unterschiedlich, so dass Wärmepflaster einer homogen hergestellten Charge eines Produktes von unterschiedlichen Anwendern von "nicht wärmend" bis "viel zu heiss" empfunden werden können. Zusätzlich verstärken eine Vielzahl von körpereigenen und externen Faktoren, wie z.B. Sport, Sauna, Wasserbetten, luftundurchlässige Kleidung, das persönliche Wärmeempfinden.

Ein weiterer Nachteil bei wirkstoffhaltigen Wärmepflastern ist, dass der bereits in die Haut des Anwenders penetrierte Wirkstoff noch geraume Zeit weiter wirkt nachdem das Pflaster entfernt wurde.

Bei Pflastern auf Basis von exothermen chemischen Reaktionen ist zum einen nachteilig, dass diese bis zum erreichen der Solltemperatur eine gewisse Vorlaufzeit benötigen. Gemäß Japanese Industrial Standard "Disposable Body Warmers" darf diese Zeit maximal 30 min bis zum Erreichen von mindestens 40 °C sein.

Ist die chemische Reaktion erst angelaufen, so lässt sie sich nicht mehr stoppen. Das Produkt ist damit nur zum einmaligen Gebrauch direkt nach Öffnen der Verpackung geeignet. Während der Anwendung zeigen die Produkte eine Schwankung in der Maximaltemperatur sowie zum Reaktionsende einen undifferenzierten Temperaturabfall. Gemäß dem Japanese Industrial Standard soll die Temperatur jedoch nach 30 min zwischen min. 40 und max. 45 °C liegen und nach 8 Stunden Anwendungsdauer nicht unter 39 °C fallen.

EP 1 782 767 A1 offenbart ein Wärmepflaster aufweisend eine Wärme entwickelnde Schicht mit einen Gehalt an Kohlenstoff Nanoröhren. Wärme wird durch eine exotherme Reaktion erzeugt.

WO 2005/044141 A2 offenbart ein Wärmepflaster aufweisend eine Wärme entwickelnde Schicht welche Kohlenstofffasern aufweist.

WO 2006/122736 A2 offenbart ein Bucky Paper zur Wärmeerzeugung. Aufgabe der Erfindung ist es ein zu den bekannten Wärmepflastern alternatives Wärmepflaster bereitzustellen, welche eine gleichmäßige und vom Verbraucher kontrollierbare Wärmeentwicklung zeigt.

In einem erfindungsgemäßen Wärmepflaster erfolgt die Wärmeentwicklung durch Widerstandsheizelemente auf Basis von Kohlenstoff Nanoröhren (Carbon Nanotubes - CNT). Ein entsprechend ausgerüstetes Pflaster erreicht nach Einschalten, dem Anlegen einer Spannung an das CNT Heizelement, innerhalb von 30 bis 180 Sekunden seine volle Wärmeleistung und kann diese dann in den Grenzen von ± 2 °C über die gesamte Anwendungsdauer konstant halten. Bevorzugt beträgt die Temperaturschwankung unter ± 1 °C.

Von Vorteil ist es, dass, sobald die Spannungsversorguung des CNT-Heizelementes unterbrochen wird, die Wärmeentwicklung endet. Durch die geringe Masse erfolgt eine schnelle Abkühlung.

Besonders vorteilhaft ist es, wenn die Wärmeleistung des CNT-Heizelementes mittels Schalter oder stufenlos mittels diskreter Bauelemente oder Halbleitern geregelt werden kann. Am einfachsten erfolgt die Einstellung über zusätzliche Schalter, in Verbindung mit Vorwiderständen, wodurch die Wärmeleistung in 2 oder mehreren Stufen, je nach persönlichem Wohlbefinden, vorgewählt werden kann.

In speziellen Ausführungsformen ist es möglich die Wärmeleistung über eine vorprogrammierte, in das Pflaster integrierte oder externe Steuerung zu regeln, so dass zum Beispiel bestimmte Temperaturprofile erreicht werden oder die Erwärmung in Intervallen erfolgt.

Funktionaler und integraler Bestandteil erfindungsgemäßer Wärmepflaster sind Heizelemente aus Kohlenstoff Nanoröhren (Carbon Nanotubes -CNT).

Die Wände der CNT bestehen wie die der Fullerene oder wie die Ebenen des Graphits nur aus Kohlenstoff, wobei die Kohlenstoffatome eine wabenartige Struktur mit Sechsecken und jeweils drei Bindungspartnern einnehmen. Der Durchmesser der Röhren liegt meist im Bereich von 1-50 nm und Längen von mehreren Millimetern für einzelne Röhren und bis zu 20 Zentimetern für Röhrenbündel wurden bereits erreicht. Je nach Detail der Struktur ist die elektrische Leitfähigkeit innerhalb der Röhre metallisch oder halbleitend; es sind auch Kohlenstoffröhren bekannt, die bei tiefen Temperaturen supraleitend sind. CNT bestehen aus reinem Kohlenstoff dessen einzelne C-Atome zu gitternetzförmigen Röhren angeordnet sind. Diese Röhrenstrukturen können dabei beidseitig offen, einseitig offen oder beidseitig geschlossen sein. Ferner können die CNTs aus Single-Wall, Double-Walled, Multi-Walled, funktionalisierte CNTs oder CNT-Bundles sowie Mischungen daraus ausgewählt werden. Erfindungsgemäß bevorzugt werden Multi-Walled CNTs.

Einzelne CNT sind hervorragende elektrische Leiter und Wärmeleiter. CNT weisen bei vergleichbarem Querschnitt eine ca. 1000-fache Strombelastbarkeit gegenüber Kupfer auf.

Die Wärmeleitfähigkeit bei Raumtemperatur von CNT ist etwa doppelt so hoch wie die des Diamanten und beträgt rund das 15-fache der Wärmeleitfähigkeit des Kupfers.

Neben anderen Herstellern werden entsprechende Multi-Walled CNT kommerziell beispielsweise unter den Namen Millenium-Tube oder Millenium-Fiber von der Firma carbon-NT&F 21, Eisenstadt, Österreich, angeboten. Ein weiterer Anbieter für CNTs ist CNT CO., LTD (Korea), die CNT unter dem Namen "C-Tube" vertreibt.

Zur Herstellung eines erfindungsgemäßen Wärmepflasters werden pulverförmige oder dispergierte, insbesondere suspendierte, CNT als Komposit in eine flächige Anwendungsform gebracht. Die CNT können dabei als dünne Schicht auf ein Substrat aufgetragen und physikalisch damit verbunden werden, z.B. als "Lackauftragung", oder durch Vermischungsprozesse homogen in ein Matrixsystem inkorporiert werden, z.B. als CNT-Polymermatrix.

Die bekannten Herstellverfahren solcher Komposite sind durch die Verwendung von CNT nicht limitiert, es können alle üblicherweise verwendeten Sprühsysteme, Auftragssysteme, Mischer, Kneter, Extruder etc. zur Anwendung kommen.

Wenn in einem solchen Komposit ausreichend viele CNT vorhanden sind, können sich diese zu einer Gitternetzstruktur, ähnlich der Struktur eines eingebetteten Vlieses, ausbilden, in dem sich die einzelne CNT berühren. Bei Stromfluss durch eine solche Gitternetzstruktur entsteht durch die an den Berührungsstellen der CNT untereinander auftretenden Kontaktwiderstände ein Spannungsabfall und damit eine Wärmeentwicklung.

Da hierbei entgegen der üblichen Widerstandsheizungen keinerlei Drahtlitzen benötigt werden, die das System versteifen, kann ein erfindungsgemäßes Wärmepflaster nicht nur sehr dünn, sondern auch sehr flexibel sein. Dies erlaubt eine hohe Formungsfreiheit der CNT Wärmepflaster sowohl in der Herstellungs- wie auch in der Anwendungsform.

Für die Verwendung von CNT in einem erfindungsgemäßen Wärmepflaster ist es von enormer Bedeutung möglichst homogen verteilte einzelne Carbon Nanotubes anstatt der in einem CNT-Pulver üblicherweise vorhandenen größeren CNT-Agglomerate zur Verfügung zu haben.

Diese Deagglomerisation kann über eine Vorbehandlung einer CNT-Dispersion vorgenommen werden, z.B. über die Behandlung per Ultraschall mittels einer Sonotrode.

Durch die so erzeugte Kavitation in der Dispersion brechen die CNT Agglomerate zu einzelnen CNT auseinander. Die Behandlungsbedingungen müssen allerdings in Abhängigkeit der verwendeten CNT sehr sorgfältig gewählt werden, da eine zu intensive Ultraschalleinwirkung zu einer Verkürzung der einzelnen Röhren führen kann.

In einem zweiten Aufbereitungsschritt werden der solcherart vorbehandelten sonotrierten CNT-Dispersion noch Verunreinigungen wie amorpher Kohlenstoff, Katalysatorreste etc. entzogen. Da diese Verunreinigungen in der Regel eine höhere Dichte aufweisen als CNT Primärpartikel können diese leicht abzentrifugiert werden. Die überstehende deagglomerisierte und aufgereinigten CNT-Dispersion kann dann direkt zur Herstellung erfindungsgemäßer CNT-Komposite eingesetzt werden.

Bei CNT-Dispersionen handelt es sich vorzugsweise um Suspensionen von festen CNT in einer flüssigen Matrix.

Der Anteil an CNT im fertigen Komposit bewegt sich üblicherweise zwischen 1 bis 15 Gew.-% bezogen auf die Gesamtmasse des Komposits.

Bevorzugt werden wasserdampfdurchlässige und/oder feuchtigkeitsaufnehmende Matrices eingesetzt. Solcherlei Matrices können Feuchtigkeitsbildung unter dem Wärmepflaster und damit mangelnde Klebkraft oder lokale Überhitzungseffekte durch Schwitzwasser verhindern.

Bevorzugt werden als Matrix Flüssigsilikone, sogenannte Liquid Silicon Rubber (LSR), wie sie handelsüblich z.B. von der Firma BAYER AG oder DOW angeboten werden, verwendet. Dabei handelt es sich um Reaktionsharzsysteme, bei denen eine Vernetzung erst nach der Vermischung von mindestens zwei Komponenten oder durch Bestrahlung startet.

Hierbei werden die deagglomerisierten und gereinigten CNT mittels Mischsystemen mit einer oder beiden LSR-Komponenten homogen vermischt, ggf. das Dispersionsmittel der CNT Deagglomerisation entfernt und die CNT / Silikonmischung durch Spritzguß, Extrusion, Gießformen, Pressen oder andere Silikonverarbeitungsarten in die für das Endprodukt gewünschte Form gebracht und diese dann zu einem festen Produkt auspolymerisiert.

Die Vernetzungsart der genutzten Silikone ist dabei für das Endprodukt unerheblich, ebenso wie die molekulare Feinstruktur der Silikone oder sonstige Additive, die je nach den Anwendungseigenschaften des finalen Wärmepflasters ausgewählt werden können.

Die Endform des CNT haltigen Komposites kann dabei durch Giessen, Spritzen, Spritzgiessen, Extrudieren, Formpressen etc. hergestellt werden. Die Schichtdicken solcher CNT/Silikon Komposite können bis zu mehreren Millimetern betragen. Bevorzugt werden jedoch Dicken zwischen 100 µm und 2500 µm, da diese eine hervorragende Flexiblität und Bruchfestigkeit aufweisen.

Erfindungsgemäß können Wärmepflaster auch durch Auftragen von CNT-haltigen Lacken oder Farben auf Substraten (Trägermaterialien) hergestellt werden. Die Lacke oder Farben können dabei wasser- oder lösemittelbasiert sein. Bei der Verarbeitung von Lacken kann der CNT Anteil dabei deutlich unter 1 Gew.-%, bezogen auf die Gesamtmasse des Lacks, liegen, was die Herstellung von dünnen homogenen CNT Schichten stark erleichtert. Nach Abdunsten des Lösemittels ist die CNT-Konzentration wieder entsprechend hoch um genug CNT-CNT-Kontakte auszubilden und damit eine Wärmeentwicklung beim Anlegen einer Spannung zu zeigen.

Die allgemeine Verarbeitung kann mit den für Lacke und Farben üblichen Technologien erfolgen, z.B. aufsprühen, aufdrucken, ausstreichen oder tauchen mit anschließendem Trocknungsvorgang und/oder Vernetzungsvorgang bei Reaktionslacken. Durch Verarbeitung von CNT-haltigen Lacken können sehr geringe funktionale Schichtdicken von ca. 10 µm bis 100 µm erreicht werden.

Bei beiden vorstehend beschriebenen Herstellungsarten von CNT Gitternetzstrukturen für Wärmepflaster wird ein elektrischer Widerstand von 1 Ohm/sq bis 100 Ohm/sq erreicht. Ohm pro square ist ein Vergleichsmaß für den Flächenwiderstand (Rsp = R mal Länge durch Breite der Probe) und läßt einen Vergleich von Einflüssen der Schichtdicken zu.

Eine weitere Form kann aus einem Gemisch aus CNT und Papierfasern bestehen welche in eine Papierform gebracht werden.

Bei einer weiteren Ausführungsform ist die Matrix in die die CNT eingebettet sind die Klebeschicht des Pflasters selbst. Aufgrund der chemischen Inertheit der CNT ist die molekulare Grundstruktur der Klebmatrix dabei unerheblich. Es können sowohl polare als auch unpolare, lösemittelhaltige als auch lösemittelfreie Schmelzhaftkleber als auch wässrige Gelmatrices (Cataplasmen) verwendet werden.

Ebenfalls ist es möglich den CNT Klebmatrices alle gängigen Arten von Additiven zur Verbesserung der finalen Produkteigenschaften zuzusetzen.

Zur Stromversorgung eines erfindungsgemäßen Wärmepflasters werden die vorstehend beschriebenen CNT Komposite zumindest an zwei nicht direkt benachbarten Punkten mit elektrischen Kontaktstellen versehen. Vorteilhaft sind zwei an gegenüberliegenden Seiten oder direkt in die CNT-Schicht integrierte elektrische Leiterbahnen. Diese Leiterbahnen können aus Metallfolien, Metalldrähten, elektrisch leitfähigen Lack oder sonstigen elektrisch leitfähigen Materialien bestehen und stellen die Anbindungspunkt der CNT zur Stromquelle her.

Die Stromquelle für ein erfindungsgemäßes Wärmepflaster wird entsprechend über weitere Leiter wie zum Beispiel Kupferkabel mit diesen Kontaktstellen verbunden, wobei der Stromfluss über einen Schalter unterbrochen bzw. aktiviert werden kann.

Besonders vorteilhaft ist es, wenn durch zusätzliche Steuerungsmöglichkeiten, wie zum Beispiel Speicherprogrammierbare Steuerungen (SPS) vorwählbare Temperaturen oder Temperaturprofile eingestellt werden können.

Erfindungsgemäße Wärmepflaster werden über eine Stromquelle versorgt, die eine Spannung von maximal 48 V, insbesondere zwischen 3 bis 12 V liefern. Damit liegen die Erfindungsgemäßen Wärmepflaster deutlich unterhalb des Bereichs von elektrisch aktiven Pflastern mit Piezoelementen, die eine Aufladungsspannung von 100 V bis 3000 V benötigen. Gegenüber den elektrisch aktiven Pflastern mittels Piezoelektrik zur Freisetzung von kosmetischen, dermatologischen oder medizinischen Wirkstoffen sind solche auf Basis erfindungsgemäßer CNT Wärmepflaster somit für den Anwender deutlich elektrisch sicherer.

Als Stromquelle können dabei z.B. handelsübliche Batterien oder wiederaufladbare Akkus oder Steckernetzteile zum Einsatz kommen. Bevorzugt sind Knopfbatterien, besonders bevorzugt flexible Flachbatterien oder- akkus, da diese sich auf Grund ihrer geringen Abmessungen leicht in das Wärmepflaster integrieren lassen. Aber auch Energiequellen in Form von Solarzellen sind möglich. Für Daueranwendungen können auch Anschlüsse an die Stromquellen von Mobiltelefonen, Autos und Motorrädern vorgesehen werden.

In der einfachsten Ausführungsform erfindungsgemäßer Wärmepflaster sind die CNT, die Leiterbahnen und die Energiequelle in eine Klebmatrix integriert, wobei die Klebmatrix einseitig mit einem nichtklebenden Trägermaterial abgedeckt ist.

Erfindungsgemäß lassen sich als Trägermaterialien alle starren und elastischen Flächengebilde aus synthetischen und natürlichen Rohstoffen eignen. Bevorzugt sind Trägermaterialien, die so eingesetzt werden können, dass sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona-behandeln und Hydrophobieren; geläufige Nachbehandlungen sind Kalandern, Tempern, Kaschieren, Stanzen und Eindecken.

In einer bevorzugten Ausführungsform der Erfindung werden als Trägermaterial Polymerfolien, Vliese, Gewebe sowie deren Kombinationen eingesetzt. Als Trägermaterialien stehen u.a. Polymere wie Polyethylen, Polypropylen und Polyurethan oder auch Naturfasern zur Auswahl.

Erfindungsgemäß besonders bevorzugt ist es ein metallocen-Polyethylen-Vliesstoff als Trägermaterial zu verwenden. Solcherlei metallocen-Polyethylen-Vliesstoffe weisen vorzugsweise folgende Eigenschaften auf:
- ein Flächengewicht von 40 bis 200 g/m², insbesondere von 60 bis 120 g/m², und/oder
- eine Dicke von 0,1 bis 0,6 mm, insbesondere von 0,2 bis 0,5, und/oder
- eine Höchstzugkraft-Dehnung längs von 400 bis 700% und/oder
- eine Höchstzugkraft-Dehnung quer von 250 bis 550%.

Auch können als Trägermaterialien bekannte Vliese eingesetzt werden, die mechanisch verfestigt sind, und zwar durch das Übernähen mit separaten Fäden oder durch das Vermaschen. Im ersten Falle ergeben sich die Vlies-Faden-Nähgewirke. Zur Herstellung dieser wird ein Faservlies vorgelegt, das beispielsweise quergetäfelt sein kann und mittels separater Fäden in Fransen- oder Trikotlegung übernäht wird. Bei der zweiten Art der Verfestigung wird ebenfalls vorzugsweise ein quergetäfeltes Vlies vorgelegt. Während des Verfestigungsvorganges ziehen Nadeln aus dem Vlies selbst Fasern heraus und formen sie zu Maschen, wobei in Fransenlegung Nähte entstehen.

Bevorzugt ist das Trägermaterial wasserdampfdurchlässig, um ein zu starkes Schwitzen der Haut während der Anwendung ableiten zu können.

Auf der Haut zugewandten Seite ist es vorteilhaft die CNT-Klebmatrix mit einer Abdeckfolie zum Schutz der klebenden Matrix vor dem Gebrauch versehen. Diese Abdeckfolie kann dabei aus allen für den Einsatzzweck gängigen Materialien bestehen und die Klebmatrix einheitlich ganzflächig oder zur besseren Entfernbarkeit aus zwei oder mehreren, ggf. überlappenden identischen oder unterschiedlichen Materialien bestehen.

In einer bevorzugten Ausführungsform ist die CNT Schicht nicht in die Klebmatrix integriert, sondern als separate Schicht zwischen der Klebmatrix und dem Trägermaterial eingearbeitet. Zur elektrischen Isolation und um mögliche Migrationen der CNTs in die Klebmatrix oder das Trägermaterial zu verhindern kann zwischen den einzelnen Schichten eine zusätzliche Sperrschicht aus einem CNT undurchlässigen Material eingearbeitet werden. Dieses CNT undurchlässige Material kann dabei das CNT Komposit auch gesamthaft in Beutelform umschließen.

Vorteilhaft ist es auch, zwischen dem CNT Komposit und dem Trägematerial eine wärmeisolierende Schicht, z.B. eine Metallfolie, einzuarbeiten.

Die Größe und äußere Form eines erfindungsgemäßen CNT Wärmepflasters ist beliebig und kann der jeweiligen Indikation angepasst werden. Zur Anwendung bei Erkrankungen des Bewegungsapparates oder zur Behandlung von Cellulite wird man eher großflächig rechteckige Formen wählen, zur Antifaltenbehandlung bzw. gezielten Freisetzung kosmetischer oder dermatologischer Wirkstoffe eher kleine, dem Anwendungsgebiet entsprechend vorgeformte Geometrien.

Zusätzlich zu dem reinen Nutzen als Wärmepflaster können erfindungsgemäße CNT Wärmepflaster auch in der Kombination mit wirkstoffhaltigen Matrices eingesetzt werden wobei die Wärme zur Verbesserung der Hautpenetration führt. Bei dieser Art der Anwendung werden die kosmetischen, medizinischen, oder dermatologischen Wirkstoffe als zum Beispiel monolitisches System in die hautzugewandte Klebmatrix eingearbeitet.

Der Wirkmechanismus von Pflastern zur Verabreichung pflegender, kosmetischer oder pharmazeutischer Substanzen in die Haut unterliegt dabei einem analogen Funktionsprinzip wie Transdermale Therapeutische Systeme (TTS).

Transdermal Therapeutische Systeme zur Abgabe von Wirkstoffen in bzw. durch die Haut sind seit langer Zeit bekannt und stellen pflasterartige, insbesondere arzneistoffdotierte Systeme dar.

Die zeitabhängige Freisetzung der Substanz, beispielsweise dem Arzneistoff, aus einem TTS erfolgt in Abhängigkeit ihres Verteilungskoeffizienten TTS/Haut und ihrer Diffusion im Bereich des TTS und der Haut.

Beide Faktoren werden durch die Zusammensetzung der Matrix bestimmt, wodurch die pro Zeiteinheit freigesetzte Menge und die Dauer der Wirksamkeit direkt beeinflusst werden können. Üblicherweise werden hierfür Hydrokolloide, Lösungsvermittler und Enhancer eingesetzt, welche eine verbesserte Löslichkeit und Diffusion sowie einen schnelleren Übergang der Substanz von TTS in die Haut ermöglichen.

Durch Erwärmung der Matrix kann die Beweglichkeit aller Matrixteilchen erhöht werden, ggf. können feste Wirkstoffe bei Anwendung verflüssigt werden. Dadurch wird die Diffusion und Penetration größerer Wirkstoffmengen pro Zeiteinheit ermöglicht.

Durch die Wärmewirkung wird die gesamte Versorgung des Hautareals verbessert, dadurch können Wirkstoffe zusätzlich ggf. tiefer in die zu behandelnden Bereiche eindringen.

In einer weiteren erfindungsgemäßen Ausführungsform eines wirkstoffhaltigen CNT Wärmepflasters können die Wirkstoffe in einem Reservoirsystem satt in einem monolithischen Matrixsystem vorliegen. Besonders vorteilhaft sind die Wirkstoffe dabei in eine feste Gelstruktur inkorporiert, z.B. in einem Gelatinegel, welches sich als separate Schicht zwischen der hautzugewandten Klebmatrix und der CNT haltigen Schicht befindet. Dies hat die Vorteile, dass das Reservoir eine sehr hohe Konzentration an Wirkstoff enthalten kann ohne die Klebkraft zu beeinflussen, die Lagerstabilität des Wirkstoffs durch eine festere Einbindung in eine Gelstruktur erhöht wird und dabei gegebenfalls Inkompatabilitäten des Wirkstoffs mit der Klebmatrix verhindert werden können, sowie dass die Anwendungs- bzw. Freisetzungsdauer eines wirkstoffhaltigen CNT Wärmepflasters verlängert werden kann.

Bei Anwendung und entsprechender Wärmeentwicklung des CNT Pflasters erweicht bzw. verflüssigt sich die Gelstruktur und der ehemals fest eingebundene Wirkstoff kann frei durch die Klebmatrix in die Haut diffundieren. Je nach Wirkstofflöslichkeit können dabei hydrophile wie lipophile Gelstrukturen zur Anwendung kommen.

Pharmazeutisch oder dermatologisch wirksame Substanzen können der Klebmatrix eine erfindungsgemäßen Wärmepflasters vorzugsweise bis zu 40 Gew.-%, besonders zu 0,1 bis 25 Gew.-%, ganz besonders zu 0,5 bis 10 Gew.-% zugesetzt sein.

Typische Wirkstoffe sind - ohne den Anspruch der Vollständigkeit im Rahmen der vorliegenden Erfindung zu erheben:

| **Indikation:** | **Wirkstoff** |
|---|---|
| Nichtsteroidale Antirheumatika | Glykolsalicylat |
| | Flufenaminsäure |
| | Ibuprofen |
| | Capsaicinoide |
| | Etofenamat |
| | Ketoprofen |
| | Piroxicam |
| | Indomethacin |
| Antipuriginosa | Polidocanol |
| | Isoprenalin |
| | Crotamiton |
| Keratolytika | Harnstoff |
| | Salicylsäure |

Erfindungsgemäß vorteilhaft sind ein oder mehrere hautpflegende und/oder kosmetische Wirkstoffe aus der Gruppe der lipophilen Zusatzstoffe, insbesondere aus folgender Gruppe:
Acetylsalicylsäure, Atropin, Azulen, Hydrocortison und dessen Derivaten, zum Beispiel Hydrocortison-17-valerat, Vitamine, zum Beispiel Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, zum Beispiel Nachtkerzenöl, Borretschöl oder Johannisbeerkernöl, Fischöle, Lebertran aber auch Ceramide und ceramidähnliche Verbindungen.

Besonders vorteilhaft werden der oder die Zusatzstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen. Besonders bevorzugter NO-Synthasehemmer ist das Nitroarginin.

Weiter vorteilhaft werden der oder die Zusatzstoffe gewählt aus der Gruppe, welche Catechine und Gallensäureester von Catechinen und wäßrige beziehungsweise organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis, des grünen Tees. Auch enthalten diese Extrakte zusätzliche vorteilhafte Inhaltsstoffe wie zum Beispiel Polyphenole beziehungsweise Catechine, Coffein, Vitamine, Zucker, Mineralien, Aminosäuren, Lipide.

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Zusatzstoff im Sinne der vorliegenden Erfindung.

Ganz besonders vorteilhaft sind die Teesorten Camellia sinenis, C. assamica, C. taliensis beziehungsweise C. irrawadiensis und Kreuzungen aus diesen mit beispielsweise Camellia japonica.

Bevorzugte Zusatzstoffe sind ferner Polyphenole beziehungsweise Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallo-catechingallat.

In einer vorteilhaften Ausführungsform ist ein erfindungsgemäßes CNT Wärmepflaster so ausgelegt, dass die wirkstoffhaltige Klebmatrix bei Bedarf manuell abgelöst und durch eine neue wirkstoffhaltige Klebmatrix ersetzt werden kann, z. B. weil sämtlicher Wirkstoff aus der Klebmatrix abgegeben wurde.

Auch bei erfindungsgemäßen CNT Wärmepflastern ohne Wirkstoff kann diese Art der Ausführung bei Mehrfachgebrauch aus hygienischen Gründen vorteilhaft sein.

Bei einer ebenfalls sehr vorteilhaften Ausführungsform eines erfindungsgemäßen CNT Wärmepflasters kann bei Dauergebrauch die Energiequelle manuell ausgetausch werden. In diesem Fall kann das CNT Wärmepflaster mehrere Tage bis Wochen ohne mechanische Hautreizung durch Entfernung und Wiederanbringung am Körper des Anwenders verbleiben.

Im umgekehrten Ausführungsfall können mehrere CNT Wärmepflaster nacheinander mit der identischen Energiequelle betrieben werden.

Die Erfindung wird im Folgenden anhand von Figuren näher erläutert.

Die Erfindung ist nicht auf das vorstehend Beschriebene und in den Zeichnungen Gezeigte beschränkt, und viele Modifikationen sind denkbar, ohne eine Abweichung vom Umfang der beigefügten Ansprüche darzustellen.

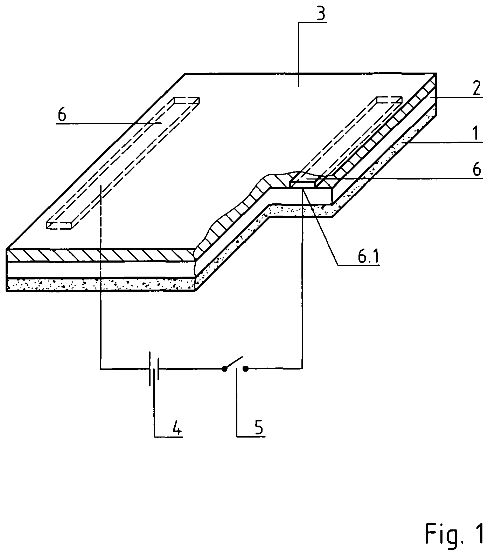

Figur 1 zeigt schematisch ein erfindungsgemäßes Wärmepflaster aufweisend eine Trägermaterial (1), eine CNT-haltige Matrixschicht (2). Die Matrixschicht (2) weist an zwei gegenüberliegenden Seiten je eine Leiterbahn (6) auf, die die elektrischen Verbindungspunkte (6.1) zur Anbindung an eine Stromquelle aufweisen. Über diese kann das Pflaster mit Hilfe von Leitungen und ggfs Schalter (5) oder Steuereinrichtung mit einer Stromquelle (4) verbunden werden. Die Matrixschicht (2) ist durch eine Klebeschicht (3) abgedeckt.

## Patentansprüche

1. Wärmepflaster aufweisend eine wärmeentwickelnde Schicht, wobei die wärmeentwickelnde Schicht einen Gehalt an Kohlenstoff Nanoröhren (Carbon Nano Tubes - CNT) aufweist, **dadurch gekennzeichnet, dass** die wärmentwickelnde Schicht aus in einer Matrix eingebetteten CNT besteht, wobei sich die CNT untereinander teilweise berühren und ein leitfähiges Netzwerk ausbilden und die wärmeentwickelnde Schicht flächig mit einem Trägermaterial verbunden ist..

2. Wärmepflaster nach Anspruch 1, **dadurch gekennzeichnet, dass** die wärmeentwickelnde Schicht CNT in einer Konzentration von 1 bis 15 Gew.-% aufweist.

3. Wärmepflaster nach mindestens einem der vorhergegen Ansprüche, **dadurch gekennzeichnet, dass** die CNT in einer Silikonmatrix, einer Lackmatrix, einer Klebmatrix, Papiermatrix eingebettet sind.

4. Wärmepflaster nach mindestens einem der vorhergegen Ansprüche, **dadurch gekennzeichnet, dass** als Trägermaterialien ein starres oder elastisches Flächengebilde aus synthetischen und natürlichen Rohstoffen verwendet wird.

5. Wärmepflaster nach mindestens einem der vorhergegen Ansprüche, **dadurch gekennzeichnet, dass** als Trägermaterial ein Polymerfolien, Gewebe, Gewirke, Gelege, Vlies, Laminat, Netz, Folie, Schäum und/oderPapier, sowie deren Kombinationen, eingesetzt wird.

6. Wärmepflaster nach mindestens einem der vorhergegen Ansprüche, **dadurch gekennzeichnet, dass** das Trägermaterial vorbehandelt und/oder nachbehandelt wird, vorzugsvweise durch Corona-behandlung und Hydrophobieren, Kalandern, Tempern, Kaschieren, Stanzen und/oder Eindecken.

7. Wärmepflaster nach mindestens einem der vorhergegen Ansprüche, **dadurch gekennzeichnet, dass** zur Stromversorgung die wärmeentwickelnde Schicht zumindest an zwei nicht direkt benachbarten Punkten mit elektrischen Kontaktstellen versehen ist, vorteilhaft durch zwei an gegenüberliegenden Seiten elektrische Leiterbahnen.

8. Wärmepflaster nach Anspruch 9, **dadurch gekennzeichnet, dass** die Leiterbahnen aus Metallfolien, Metalldrähten, elektrisch leitfähigen Lack oder sonstigen elektrisch leitfähigen Materialien bestehen,

9. Wärmepflaster nach mindestens einem der vorhergegen Ansprüche, **dadurch gekennzeichnet, dass** die CNT, die Leiterbahnen und die Energiequelle in eine Klebmatrix integriert sind, wobei die Klebmatrix einseitig mit einem nichtklebenden Trägermaterial abgedeckt ist.

10. Wärmepflaster nach Anspruch 7, **dadurch gekennzeichnet, dass** das Trägermaterial Polyethylen, Polypropylen und Polyurethan oder auch Naturfasern enthält, insbesondere ein metallocen-Polyethylen-Vliesstoff ist.

11. Wärmepflaster nach mindestens einem der vorhergegen Ansprüche, **dadurch gekennzeichnet, dass** die Klebmatrix pharmazeutisch oder dermatologisch wirksame Substanzen aufweist, vorzugsweise bis zu 40 Gew.-%, besonders bevorzugt 0,1 bis 25 Gew.-%, ganz besonders bevorzugt zu 0,5 bis 10 Gew.-%.

## Claims

1. Hot compress having a heat-developing layer, wherein the heat-developing layer has a content of carbon nanotubes (CNTs), **characterized in that** the heat-developing layer consists of CNTs embedded in a matrix, wherein the CNTs can in part contact one another and form a conducting network and the heat-developing layer is surface joined to a support material.

2. Hot compress according to Claim 1, **characterized in that** the heat-developing layer has CNTs at a concentration of 1 to 15% by weight.

3. Hot compress according to at least one of the preceding claims, **characterized in that** the CNTs are embedded in a silicone matrix, a paint matrix, an adhesive matrix, paper matrix.

4. Hot compress according to at least one of the preceding claims, **characterized in that**, as support materials, a rigid or elastic flat material made of synthetic and natural raw materials is used.

5. Hot compress according to at least one of the preceding claims, **characterized in that** the support material used is a polymer film, woven fabric, knitted fabric, laid fabric, nonwoven, laminate, net, film, foam and/or paper, and combinations thereof.

6. Hot compress according to at least one of the preceding claims, **characterized in that** the support material is pretreated and/or post-treated, preferably by corona treatment and hydrophobizing, calendering, tempering, laminating, stamping and/or covering.

7. Hot compress according to at least one of the preceding claims, **characterized in that**, for power supply, the heat-developing layer is provided with electrical contact sites at least at two points that are not directly adjacent, advantageously by two electrical conductive webs at opposite sides.

8. Hot compress according to Claim 9, **characterized in that** the conductive webs consist of metal films, metal wires, electrically conducting paint or other electrically conductive materials.

9. Hot compress according to at least one of the preceding claims, **characterized in that** the CNTs, the conductive webs and the energy source are integrated into an adhesive matrix, wherein the adhesive matrix is covered at one end with a non-adhesive support material.

10. Hot compress according to Claim 7, **characterized in that** the support material consists of polyethylene, polypropylene and polyurethane, or else natural fibers, and is in particular a metallocene-polyethylene-nonwoven material.

11. Hot compress according to at least one of the preceding claims, **characterized in that** the adhesive matrix comprises pharmaceutically or dermatologically active substances, preferably up to 40% by weight, particularly preferably 0.1 to 25% by weight, very particularly preferably at 0.5 to 10% by weight.

## Revendications

1. Pansement chauffant présentant une couche dégageant de la chaleur, la couche dégageant de la chaleur présentant une teneur en nanotubes de carbone (Carbon Nano Tubes - CNT), **caractérisé en ce que** la couche dégageant de la chaleur est constituée par des CNT incorporés dans une matrice, les CNT étant partiellement en contact les uns avec les autres et formant un réseau conducteur et la couche dégageant de la chaleur étant reliée de manière surfacique avec un matériau support.

2. Pansement chauffant selon la revendication 1, **caractérisé en ce que** la couche dégageant de la chaleur contient des CNT en une concentration de 1 à 15% en poids.

3. Pansement chauffant selon au moins l'une quelconque des revendications précédentes, **caractérisé en ce que** les CNT sont incorporés dans une matrice de silicone, une matrice de laque, une matrice adhésive, une matrice de papier.

4. Pansement chauffant selon au moins l'une quelconque des revendications précédentes, **caractérisé en ce qu'**on utilise, comme matériaux support, une structure plane rigide ou élastique en matières premières synthétiques et naturelles.

5. Pansement chauffant selon au moins l'une quelconque des revendications précédentes, **caractérisé en ce qu'**on utilise comme matériau support une feuille en polymère, un tissu, un tricot, une nappe, un non-tissé, un stratifié, une toile, une feuille, une mousse et/ou un papier, ainsi que leurs combinaisons.

6. Pansement chauffant selon au moins l'une quelconque des revendications précédentes, **caractérisé en ce que** le matériau support est prétraité et/ou posttraité, de préférence par un traitement au corona et une hydrofugation, un calandrage, un traitement thermique, un contrecollage, un découpage et/ou un recouvrement.

7. Pansement chauffant selon au moins l'une quelconque des revendications précédentes, **caractérisé en ce que** la couche dégageant de la chaleur, pour l'alimentation en courant, est pourvue de sites de contact électrique en au moins deux points non directement adjacents, avantageusement de deux pistes conductrices électriques sur des faces opposées.

8. Pansement chauffant selon la revendication 9, **caractérisé en ce que** les pistes conductrices sont constituées par des feuilles métalliques, des fils métalliques, une laque électriquement conductrice ou d'autres matériaux électriquement conducteurs.

9. Pansement chauffant selon au moins l'une quelconque des revendications précédentes, **caractérisé en ce que** les CNT, les pistes conductrices et la source d'énergie sont intégrés dans une matrice adhésive, la matrice adhésive étant revêtue sur une face d'un matériau support non adhésif.

10. Pansement chauffant selon la revendication 7, **caractérisé en ce que** le matériau support contient du polyéthylène, du polypropylène et du polyuréthane ou également des fibres naturelles et est en particulier un non-tissé en métallocène-polyéthylène.

11. Pansement chauffant selon au moins l'une quelconque des revendications précédentes, **caractérisé en ce que** la matrice adhésive présente des substances pharmaceutiquement ou dermatologiquement actives, de préférence jusqu'à 40% en poids, de manière particulièrement préférée à raison de 0,1 à 25% en poids, de manière tout particulièrement préférée à raison de 0,5 à 10% en poids.
